# EUROPEAN PATENT SPECIFICATION

(11) **EP 2 787 855 B1**
(45) Date of publication and mention of the grant of the patent: **14.08.2019**
(21) Application number: 12806634.7
(22) Date of filing: 10.12.2012
(51) Int. Cl.: A43B 17/00, A43B 7/14

(54) **FOOTWEAR/INSOLE FOR FOOTWEAR**
SCHUH/EINLEGESOHLE FÜR DEN SCHUH
CHAUSSURE/SEMELLE POUR CHAUSSURE

(30) Priority: 08.12.2011 GB 201121142; 09.03.2012 GB 201204153; 30.05.2012 GB 201209615
(43) Date of publication of application: 15.10.2014
(73) Proprietor: Footjacks Ltd, Radlett, Hertfordshire WD7 8DF (GB)
(72) Inventor: STERN, Mark, Radlett, Hertfordshire WD7 8DF (GB)
(74) Representative: Boult Wade Tennant LLP
(86) International application number: PCT/GB2012/053073
(87) International publication number: WO 2013/084008

(56) References cited:
- EP-A1- 1 475 006
- EP-A1- 1 475 006
- EP-A1- 1 714 624
- EP-A1- 1 714 624
- WO-A1-00/70984
- WO-A1-00/70984
- WO-A1-2006/133382
- WO-A1-2006/133382
- FR-A1- 2 858 525
- FR-A1- 2 858 525
- US-A- 4 955 148
- US-A- 4 955 148
- US-A- 5 799 414
- US-A- 5 799 414
- US-A- 6 000 147
- US-A- 6 000 147
- US-A1- 2006 107 553
- US-A1- 2006 107 553
- US-A1- 2006 288 613
- US-A1- 2006 288 613
- US-A1- 2007 271 819
- US-A1- 2007 271 819

## Description

The present invention relates to footwear for treating pronation. The invention may comprise an item such as an insole which is insertable into pre-existing footwear or it may be embodied as an integral part of an item of footwear itself.

Pronation is a common foot problem. It occurs particularly in people with flat feel (Pes Planus). Pronation tends to occur in such people as they walk and refers to the condition where parts of the hind foot, mid foot and forefoot tend to lean medially (inwards) and the arch of the foot flattens and tips over medially. When this happens the soft tissues become stretched and the joint surfaces of the bones in the foot are placed at unnatural angles to one another. The tipping over of the arch leads to a decrease in the support of the structures supported by that foot.

US5799414 A1 discloses a shoe insert that can be customized by the user to control pronation of the foot and to relieve or reduce stress at painful areas of the foot comprising a film of plastic capable of being deformed to the shape of the foot by the weight of the user. Various insoles for treating over pronation are known. Some of these include a built up or raised area beneath the arch of the foot to contact and push upwardly on the arch. However, many people still find such insoles ineffective in treating the problem.

Insoles are also known which have parts of the arch area removed. However, this has been found to be ineffective in stopping pronation when a person walks and each step begins with a heel strike.

The present invention provides a footbed as set out in claim 1

In one embodiment, the footbed is formed with a void in the heel region, an area referred to herein as V1, positioned beneath the lateral aspect of the anterio-medial ground contact of the calcaneus, being lateral to a midline of the foot and aligned substantially with the 4^{th} toe. Most text books on the subject provide that the anterior aspect of the calcaneus is not in ground contact. The writer has found, however, that this ground contact does occur in people with Pes Planus, and in conjunction with others, allows pronation to occur. The weight of the body reaches this point *before* the anterior arch abutments contact the ground. At this point the arches are formed loosely with flexibility between the joints. Once the posterior abutments at the Tuber Calcanei and the anterior abutments are in ground contact with the weight of the body acting through the arches, ground reaction forces 'firm up' the arches.

With pronation, the anterio-medial calcaneal ground contact functionally acts as an abutment to both medial and lateral long plantar arches. Instability at this contact point allows the arches to 'tip over' provided either the anterior or posterior medial calcaneal abutments are allowed to roll medially. This can be prevented in a flat shoe, with a void in the lateral heel which tips and holds the anterio-medial calcaneal contact laterally or with a void, beneath the medial process of the tuber calcanei (referred to herein as area V3) or a combination of voids in these areas.

A void directly beneath the lateral aspect of the anterio-medial calcaneal ground contact (V1) serves to prevent pronation at the talocalcaneonavicular (TCN) joint complex which occurs with a heel strike when the weight of the body reaches the ground contact point.

In alternatives described below, the body may be formed with voids in those parts of the lateral heel which are not within the region V1 described above and which indirectly prevent medial rolling at the anterio-medial calcaneal ground contact. For example, the body may be formed with voids under the lateral process of the tuber calcanei (V2), and under the anterio-lateral calcaneal ground contact (V4) occurring in people with collapsed arches.

In addition to the aforementioned, voids may be beneath any, a combination, or all of the metatarsal joints (V5-V9) and/or under the tuberosity beneath the distal phalanx of the hallux (V10).

The footbed may also include a raised mound in the medial arch area of the midfoot and at least one void anywhere in the arch area of the midfoot. Alternatively, the footbed may be substantially flat and have at least one void formed in the medial longitudinal arch area, or in the lateral longitudinal arch area of the midfoot (i.e. regions 14b and 14c as described further below).

The voids should preferably be separate and beneath each individual area described above as these increase the resistance to pronation.

The upper surface of the footbed may be arranged to support the foot with a slope downwardly from the heel region to the toe region at an angle of less than 20°, for flat or low-heeled footwear.

In another example below, for higher-heeled footwear, the footbed is arranged to support the foot so that it slopes down from the heel region to the toe region at an angle of or greater than 20°. At this angle there is no pronation permitted by the TCN joint complex through the mid foot. However, inversion and eversion of the foot occurs as a consequence of rolling medially or laterally across the metatarsal and phalangeal ground contacts and at the heel contact. To prevent this, the footbed may further comprise a void beneath the anterio-medial ground contact which extends both medially and laterally of the midline. The or each void in the footbed may comprise a hole through the footbed or a depression in the footbed. Further, a thin, flexible layer may extend over the or each void to cover the void, but providing no support to the foot. The layer must be deformable into the or each void under the weight of a person standing on the footbed.

The or each void has a minimum size of approximately 0.6 cm² to 1 cm², allowing the tissues in the sole of the foot to sink into the void.

The footbed may comprise a body made up of a plurality of separate bodies, which may be releasably connectable to each other. The footbed may be formed integrally with an item of footwear, and the item of footwear may comprise a flexible sock or stocking. Alternatively, the footbed may be releasably attachable to the sole of a foot. In a further alternative, the footbed may comprise an insole insertable into pre-existing footwear.

The footbed may comprise one or more optionally removable sections in order to create one or more voids.

The footbed may comprise a very thin layer of particulate material at least beneath those points which directly or indirectly stabilise the abutments to the medial and longitudinal arches of the foot. The layer should be thin enough so that the particulates do not compact into a hard surface. In this way, they create voids which act at the relevant points.

The invention will now be described in more detail, by way of example only, with reference to the accompanying drawings in which:
Figure 1a is a schematic medial view of the plantar arch of a foot;
Figure 1b is a diagram illustrating the main regions of the sole of a foot;
Figure 2 illustrates a footbed in accordance with a first embodiment of the present invention;
Figure 3 illustrates a footbed in accordance with a second embodiment of the present invention;

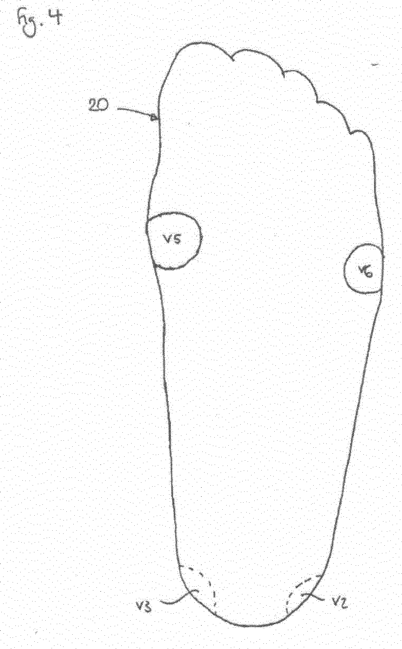

Figure 4 illustrates another footbed;
Figure 5 illustrates a footbed in accordance with the present invention and formed of separate parts (with the voids omitted for clarity); and
Figure 6 illustrates a sock incorporating a footbed in accordance with the present invention (with the voids omitted for clarity).

The human foot is a complicated structure which has many functions. One function is to support the weight of the body so that all structures above the foot are maintained in their correct positions during movement and in the standing position.

The weight of the body is supported on two longitudinal arches in the foot, the medial and longitudinal plantar arches. These arches are bony in structure and maintained by ligamentous and muscular factors. These arches have abutments to support the arches.

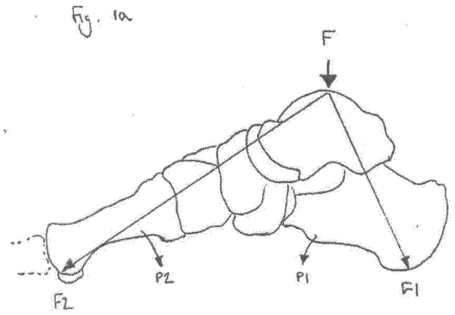

Figure 1a shows a schematic medial view of a foot, with some of the bones removed for clarity. The foot should naturally assume an arched form as shown, with the weight of the body represented by arrow F acting downwardly and then being transmitted in directions F1 and F2 to the correct ground contact points, which act as abutments to the arches. The correct posterior abutments to the arches are the medial and lateral processes of the tuber calcanei. However, in a person whose foot tends to pronate, the arch tends to collapse in the direction of arrows P1 and P2, flattening and reducing the size of the arch. Thus, with flat feet (Pes Planus), a functional abutment occurs when there is an anterio-medial and anterio-lateral calcaneal ground contact. These functional abutments have the effect of shortening the medial long plantar arch. It is the anterio-medial calcaneal ground contact which must be stabilised to prevent pronation and this can be done directly or indirectly.

Thus the abutments of the medial and lateral longitudinal arches are found in the calcaneus and under the heads of the metatarsals, although the 1^{st} and 5^{th} metatarsal heads bear most of the weight passing through the anterior aspect of the arches until the weight of the body moves into the mid foot.

Direct removal of support from beneath the lateral aspect of the anterio-medial ground contact forming a functional abutment stabilises the rear abutment at this contact and allows the longitudinal arches of the foot to firm up and function correctly during a heel strike. Indirectly stabilising the anterio-medial ground contact with a void at V3 or in the lateral heel other than in area V1 stabilises the heel until the heel starts to lift at the position the void is placed so that it ceases to "act". A void in area V3 stabilises the midfoot whilst voids in the lateral heel area other than area V1 only stabilise the midfoot in conjuction with one or more voids in the midfoot.

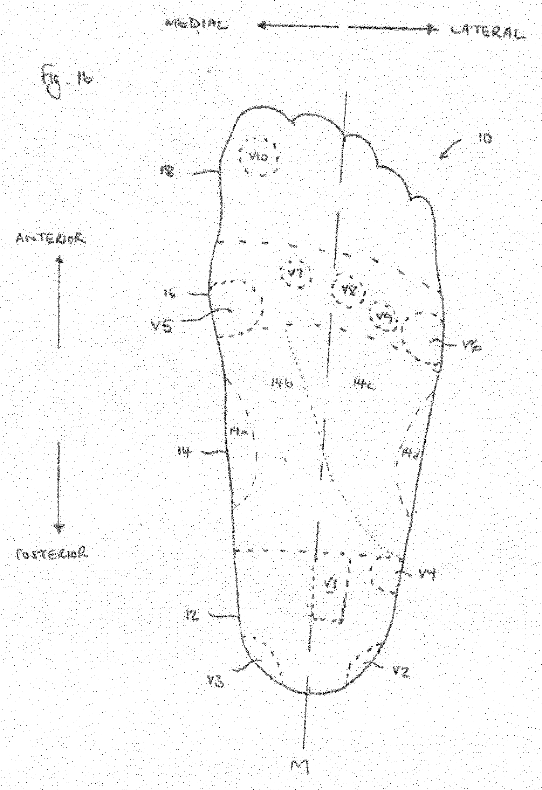

Figure 1b illustrates schematically the sole of a foot 10 which has been divided up into various regions as defined below. In the following description the terms anterior and posterior are used to denote forward and backward directions as shown by the arrows in Figure 1b, and terms medial and lateral are used to denote inner and outer directions, either side of the foot's midline, M, as shown by the arrows in Figure 1b.

For the purpose of describing the present invention, the foot in a person with Pes Planus is considered as comprising the following general regions: the heel 12, the arch 14, the heads of the metatarsal joints 16 and the toes 18. The various parts of the foot can also be described as the forefoot, consisting of the metatarsal joint region 16 and the toes 16, the mid-foot consisting of the arch 14, and the hind-foot consisting of the heel region 12. Although the heel normally functions as part of the bony arch of the foot, with hind foot pronation the anterio-medial ground contact of the calcaneus forms a functional abutment and the arch is confined to the mid foot.

The arch 14 may be considered to consist of a medial strip 14a at the extreme medial edge of the arch 14, a medial longitudinal arch region 14b, a lateral longitudinal arch region 14c and a lateral strip 14d at the extreme lateral edge. In the area of the arch 14, the notional dividing line between the medial and lateral longitudinal arch regions is somewhat diagonal as illustrated, rather than strictly following the overall midline M of the foot.

For the purpose of describing the present invention, the foot is shown with those areas (V1-V10) in the heel, the midfoot and the forefoot which stabilise the medial and lateral longitudinal arches of the foot. The illustration is schematic and the precise locations will depend on the anatomy of an individual.

V1 represents the area which stabilizes that part of the anterio-medial calcaneal ground contact which prevents pronation in the heel and through the midfoot. It is the part of the ground contact from the midline of the foot and lateral to the midline, substantially aligned with the 4^{th} toe as shown in the diagram. The area V1 is only lateral to the midline and must not extend across the midline into the medial side of the foot or have a balancing void in the medial aspect of the heel other than at V3 (defined below).

V2 is under the lateral process of the tuber calcanei.

V3 is under the medial process of the tuber calcanei.

V4 is under the anterio-lateral ground contact of the calcaneus.

V5 is under the sesamoid bones under the head of the 1^{st} metatarsal.

V6 is under the head of the 5^{th} metatarsal.

V7, V8 and V9 are under the heads of the 2^{nd}, 3^{rd} and 4^{th} metatarsals.

V10 is under the distal phalanx of the hallux.

What follows refers to the situation when the angle of the footbed, i.e. a surface on which the foot rests from hind foot to forefoot, is below approximately 20 degrees, when the foot is in the easy standing position. Below this angle the TCN joint complex is free to allow pronation through the hind and mid foot. Obviously, even with such a footbed, a slope of greater than 20 degrees occurs at push off as the heel is lifted off the ground.

The inventor has discovered that, in people whose feet pronate, the front of the calcaneus collapses anteriorly giving rise to two anterior calcaneal ground contacts. The more medial contact is roughly in line with the 3rd and 4th toes extending into the lateral half of the heel. The more lateral ground contact is on the inferior surface of the anterio-lateral calcaneal process. It is the anterio-medial calcaneal ground contact which is largely responsible for pronation through the heel and needs to be stabilised.

If a void is at V3 and not V1, then voids at V2 and V4 prevent small amounts of pronation through the lateral heel. A void at V1 itself will prevent pronation through the hindfoot with a heel strike and through the midfoot.

As mentioned, the front ground supports in the easy standing position are mainly on the tuberosities under the heads of the 1^{st} and 5^{th} metatarsals (V5 and V6) although the other metatarsal heads bear more weight once the weight of the body moves into the anterior mid foot. They represent the anterior abutments for the medial and lateral long plantar arches. The two sesamoid bones will bear the weight at the 1^{st} metatarsal head support. The 1^{st} and 5^{th} ground supports should contact the ground together.

The inventor has discovered that removal of the footbed beneath these anterior ground contact points prevents medial rolling through the forefoot. Removal of the footbed beneath the 1^{st} and 5^{th} metatarsal head contact points V5 and V6 will substantially remove pronation through the forefoot. Combinations with the other metatarsal heads can improve the effect.

The degree to which pronation is resisted can be judged by the correct lifting of the arches of the foot, the correct simultaneous striking of the 1^{st} and 5^{th} metatarsal heads on the footbed with no shifting of support towards the 1^{st} metatarsal support. There should be no medial rolling through the heel, the midfoot or the forefoot. The knee should not collapse inwards and provided all joints are reasonably free, the pelvis and shoulders should be level.

The voids beneath the abutment ground contacts as described should be large enough for the tissues of the sole of the foot to sink into them and should provide a sufficient medial contact surface along the void's medial edge to resist medial roll and divert foot motion in an anterior direction. To this end, rectangular or square voids are much better than circular voids, which do not provide as great a surface area in contact with the tissues of the sole of the foot to resist medial roll and direct foot motion in an anterior direction. Circular voids can work if the sides of the void are vertical and made with a material that resists movement of the foot. Complete removal of the footbed beneath an entire region of the foot e.g. the entire midfoot of a strip across the entire foot, has a much reduced effect compared with separate individual voids. Thus, a rectangular void is ideal with the long edge of the rectangle placed in an anterior-posterior direction. Chamferred internal edges are not advised as these provide a surface for the foot to roll along. Hook and loop material is not advisable as its surface is too firm and destroys the effect. Individual small voids multiply the resistive effect.

In the following description of embodiments of the invention, the term footbed refers to a body of material with an upper surface on which a foot rests in use. The body may constitute the base of a shoe or other item of footwear, and thus be formed as an integral part of the footwear, or it may take the form of a separate insole which can be inserted into a pre-existing item of footwear or directly attachable to the sole of the foot, for example by suction or releasable adhesive. The term insole encompasses a unitary item insertable into existing footwear to form at least part of the footbed, or a plurality of separate elements insertable into or attachable to an item of footwear to form at least part of the footbed. The separate elements may be releasably connectable to each other. The various areas of the footbed will be described using the same terms and reference numbers as the parts of the foot which overlie them in use. Thus, the heel region 12 of the footbed refers to the part which will be beneath the heel region 12 of the foot illustrated in Figure 1, and so on.

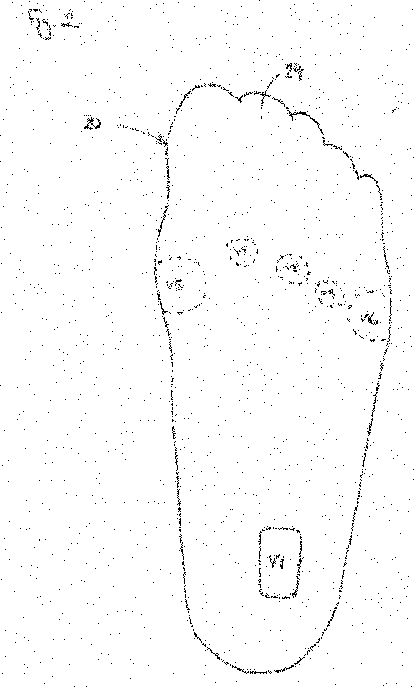

Figure 2 shows a plan view of a footbed 20 in accordance with a first embodiment of the present invention. The footbed 20 is generally flat, but has a portion removed to leave a void in the area V1. The void at V1 is within the lateral heel region beneath the lateral part of the anterio-medial calcaneal ground contact which extends into the lateral half of the heel. The void at V1 should have a minimum size of about 0.6 cm² but can be all or part of the region V1 illustrated in Figure 2. In the direction across the footbed, the void at V1 extends from the midline of the foot, laterally to a point which is substantially aligned with the lateral aspect of the 4^{th} phalange. Thus, the void at V1 is substantially aligned with the 4^{th} phalange (4^{th} toe). In use the void at V1 removes contact with that part of the anterio-medial calcaneal contact which allows pronation. It is important that the void V1 does not extend across the midline into the medial side, and that there is no balancing void to V1 on the medial side of the calcaneus (other than at area V3) as such a void negates the effect of a void at V1. A void V3 will resist a balancing void placed in the medial heel which would otherwise negate the effect achieved by a void in area V1.

Without pronation the arch of the foot is able to properly firm up and the weight of the body is able to correctly move into the mid foot as the gait cycle progresses. A roughly equal contact with the footbed under the heads of the 1^{st} and 5^{th} metatarsal joints gives equal support to forward transferred weight; this does not occur with pronation.

The presence of void at V1 controls pronation until weight reaches the middle of the mid foot and the heel starts to lift. At this point weight is transferred through the anterio aspect of the medial and lateral longitudinal arches and pronation can occur through the anterior abutments under the heads of the metatarsals. Voids in the footbed under areas V5 and any, or a combination, or all of areas V6, V7, V8, V9 will stop this occurring.

This is illustrated by the dotted lines in Figure 2 which shows optional voids at V5 beneath the sesamoid bones beneath the head of the 1^{st} metatarsal, and beneath any, or a combination, or all of V6 (beneath the tuberosity under the head of the 5^{th} metatarsal), V7, V8 and V9 (beneath the heads of the metatarsals 2, 3, and 4).

Voids under areas V7, V8 and V9 can be extended forwards to lower or remove the footbed beneath the 2^{nd} to the 5th toes. This is helpful to remove pressure for people with hammer toes, while preserving a void under the 2^{nd} to 4th metatarsals.

The void at V1 (and voids in any of the other areas as described above and further below) may be formed by complete removal of material so that there is a hole all the way through the footbed 20 from top to bottom. Alternatively, the void V1 may be formed as a depression in the upper surface 24 of the footbed 20, so that the upper surface 24 dips down in a certain area below the level of the remaining area. The tissues of the sole of the foot preferably should not be able to contact the floor of the void which can then provide a surface for the foot to roll upon depending on the sides of the void. The void can operate to tip the foot in a certain direction, to resist movement by contact of the tissues of the foot with an edge of the void, or by simply removing a surface upon which the tissues of the foot can roll.

In another alternative, the upper surface 24 comprises a thin, flexible layer which is continuous and extends over the void at V1 (or any other void) so that the void is not visible, and in order to prevent dirt accumulating in the void. However, such a cover layer must be sufficiently thin and pliable so that it does not itself support the foot and the soft tissues of the foot are still able to deform into the void.

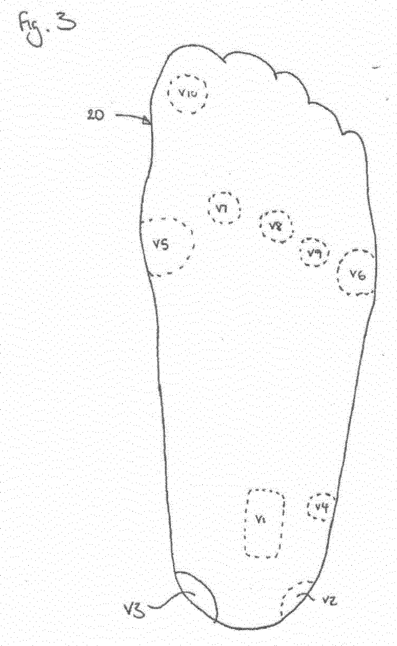

Figure 3 illustrates a plan view of a footbed 20 in accordance with another embodiment of the present invention. In this example, the footbed 20 is again generally flat and a void is provided at area V3 under the medial process of the tuber calcanei. In addition, there may be voids at any, or a combination, or all of the ground contacts at the following points: V1 (see before), V2 (lateral process of the tuber calcanei), V4 (anterio-lateral calcaneal contact), V5, V6, V7, V8 and V9 (as described before). These optional voids at V1, V2, and V4-V9 are shown in dotted lines.

Optionally a void may also be provided in a central distal portion V10 of the hallux 18a, which is shown in dotted lines, as the distal phalanx of the Hallux provides a proximal force helping to stabilise the anterior abutment of the medial longitudinal arch.

In a further embodiment where the footbed 20 is generally flat, in addition to voids in either or both of the lateral heel and in area V3, one or more further voids may be provided in the medial longitudinal arch area 14b or the lateral longitudinal arch area 14c. If the footbed 20 has a raised mound in the medial arch area, then one or more voids may be provided anywhere in the arch areas 14a-14d.

The various embodiments described above relate to a footbed where the supporting parts at the heel and the forefoot areas are substantially on a level with each other, or there is a downward slope from heel to forefoot of about 20° or less to the horizontal. Thus, this equates to substantially flat or low-heeled footwear. In such footwear the mid tarsal joints are free and pronation can occur through the mid foot, unless prevented by the features of the present invention.

However, in footwear with higher heels, where the footbed slopes from the heel to the forefoot at an angle of above about 20°, or the footwear is shaped so as to support the foot at such an angle, the arch of the foot is fully firmed up and the mid tarsals are locked. Movement at the TCN joint complex allows rolling through the forefoot and hind foot. Such rolling occurs about three pivot points, that is where the forefoot contacts the footbed, at the contact of the heel with the footbed which in turn acts on the ground via the heel of the shoe, and upwardly at the person's hip. Movement about these three points can result in inversion, where the sole of the foot tends to move towards the median plane, and eversion, where the sole of the foot tends to move away from the median plane.

Since the arch of the foot is raised, the lateral longitudinal arch is unable to prevent excessive inversion through the mid foot making it much easier to "turn" the ankle. This is particularly true with stiletto heeled footwear where the pivoting of the heel of the shoe is on a very fine point. From full inversion to full eversion, the knee may be moving through nearly 180°, much more than when the footbed is substantially flat.

Thus with high heels the anterio-medial and lateral arch abutments must be stabilised. Any "rolling" of the foot at footbed contact will cause a shift of body weight on the shoe. With a stiletto heel this will make it much more likely that a complete tipping over of the shoe will occur giving rise to a "turning" of the ankle medially or laterally.

Stabilisation of the anterior abutments requires voids at V5 (under the 1^{st} metatarsal head under the sesamoid bones) and at V6 (under the tuberosity under the head of the 5^{th} metatarsal). Voids (V7, V8, V9) under heads of the metatarsals 2-4 also reduce rolling. Voids under the metatarsals should preferably be separate voids and not one joined up void unless the sides of the void are vertical.

The anterio-medial ground contact (VI plus ground contact crossing into the medial calcaneus) and areas V2 and V3 are the key calcaneal contact points which allow rolling at the heel as they are the posterior abutments, actual and functional. Voids beneath all three points provide maximal resistance to heel rolling. A void under the medial aspect of the anterio-medial calcaneal bony ground contact prevents medial rolling as it is on the medial half of the foot. A void under the lateral aspect of the same contact prevents lateral rolling being on the lateral half of the foot. A void under both aspects will reduce rolling both ways. Preferably the void should be not much bigger than the area of the ground contact as larger voids will permit a degree of rolling.

A void beneath the anterio-medial bony calcaneal ground contact substantially reduces medial and lateral rolling whilst a void only at V3 will reduce rolling medially, one only at V2 will reduce rolling laterally.

Stabilising both anterior and posterior abutments has the maximum effect in reducing rolling. Stabilising either the front or the rear abutments separately will have some effect on reducing rolling at the other.

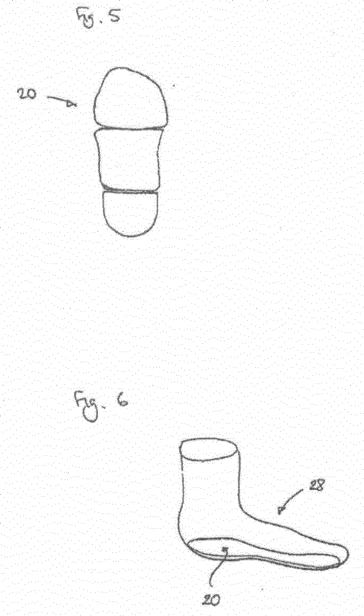

In the various embodiments described above, the footbed 20 may comprise a single body of material, or it may consist of a number of separate bodies of material. These separate bodies may be releasably connectable to one another. The bodies may be connectable directly to each other as shown in Figure 5, or spaced from each other but connected by means of straps or struts. The releasable connection may be by means of hook and loop fasteners or any other convenient fastening means. The releasable interconnection allows a footbed to be tailored to a particular user by combining and interchanging footbed parts of different configurations.

The footbed 20 may take the form of a separate insole which can be inserted into pre-existing footwear.

Alternatively, the footbed may be formed integrally into an item of footwear such as a shoe, boot or sandal. The footbed 20 may also be formed integrally with a flexible sock 28 or stocking as shown in Figure 6.

The user could then simply wear existing footwear over the sock 28. Alternatively, the sock itself could constitute an item of footwear in its own right. This could be worn indoors in the manner of known "slipper socks" (which are simply socks with anti-slip material on the lower surface) or, if made from suitably durable, waterproof material it could be worn for outdoor use.

Thus, the present invention provides an improved footbed which alters the support provided to a foot in order to halt pronation. It will be appreciated that the precise configuration of the footbed may take many forms and the details can be varied from those shown in the attached drawings without departing from the scope of the claims. Further optional features may also be provided.

For example, the footbed 20, whether separate or integrally formed with an item of footwear, may initially be provided with a continuous upper surface 24 with sections which are optionally removable to create one or more of the voids as discussed above so that the user, having purchased the footbed 20, can remove portions as required to give a choice of possible configurations.

It will also be appreciated that while a single void is illustrated in each of the various areas of V1-V10, each area may in fact include more than one void. The embodiments above also cover the situation where the footbed 20 is built up around the ground contact points described so as to leave a void at the claimed points, sufficient to prevent pronation.

The footbed can be formed by a very thin layer of particulate material, at least at the bony ground contact points in the heel and under the metatarsals as previously described. When a person stands on such a footbed, the particulate naturally moves under the person's weight in order to create voids in the necessary areas. The layer should be thin enough that it does not compact into a solid firm layer for the effect to work.

## Claims

1. A footbed configured for supporting a foot and including a void or voids formed in the footbed, beneath all of each of the following regions (i) and (ii) of the footbed, wherein the or each void has a minimum size of 0.6 cm² and configured so that the tissues of the foot deform into the void or voids, **characterized in that**:
(i) is a first region located on the footbed to underlie a lateral part of an anterio-medial ground contact portion of a calcaneus of the foot when supported by the footbed and situated in the anterior aspect of the calcaneus, having a medial border along a longitudinal midline of the heel and extending laterally of the midline to a point which is substantially aligned with the lateral aspect of the 4^{th} phalange; and
(ii) is a second region located on the footbed to underlie an anterio-lateral ground contact portion of the calcaneus on the inferior surface of the anterio-lateral calcaneal process, in an anterio-lateral region of the calcaneus;
wherein the void or voids of the first region and the second region do not cross medially of the longitudinal midline of the heel and with no other balancing voids medial of the midline of the heel except for voids located on the footbed to underlie the medial process of the tuber calcanei of the foot.

2. A footbed as claimed in claim 1, wherein the footbed comprises a plurality of separate bodies.

3. A footbed as claimed in claim 2, wherein the separate bodies are releasably connectable to each other.

4. A footbed as claimed in claim 1, wherein the footbed is integral with an item of footwear.

5. A footbed as claimed in claim 4, wherein the item of footwear comprises a flexible sock or stocking.

6. A footbed as claimed in claim 1, wherein the footbed is configured to be releasably attachable to a sole of a foot.

7. A footbed as claimed in claim 1, wherein the footbed comprises an insole insertable into pre-existing footwear.

8. A footbed as claimed in claim 1, wherein the footbed comprises one or more optionally removable sections in order to create one or more voids.

9. A footbed as claimed in claim 1, wherein each void comprises a hole through the footbed or a depression in the footbed.

10. A footbed as claimed in claim 9, further comprising a thin, flexible layer which extends over each void and is configured to be deformable into each void under the weight of a person standing on the footbed.

11. A footbed as claimed in any preceding claim, wherein the void or voids in the first and second regions are created by the use of particulate material.

## Patentansprüche

1. Fußbett, das zum Stützen eines Fußes konfiguriert ist und einen Hohlraum oder Hohlräume einschließt, die in dem Fußbett jeweils unterhalb von allen der folgenden Bereiche (i) und (ii) des Fußbetts ausgebildet sind, wobei der oder jeder Hohlraum eine Mindestgröße von 0,6 cm2 aufweist und so konfiguriert ist, dass sich die Gewebe des Fußes in den Hohlraum oder die Hohlräume verformen, **dadurch gekennzeichnet, dass**:
(i) ein erster Bereich ist, der sich auf dem Fußbett befindet, um einen seitlichen Teil eines anterio-medialen Bodenkontaktabschnitts eines Fersenbeins des Fußes zu unterlegen, wenn er von dem Fußbett gestützt wird, und sich im anterioren Aspekt des Fersenbeins befindet, mit einer medialen Grenze entlang einer Längsmittellinie der Ferse und sich seitlich der Mittellinie bis zu einem Punkt erstreckend, der im Wesentlichen mit dem lateralen Aspekt der 4. Phalanx ausgerichtet ist; und
(ii) ein zweiter Bereich ist, der sich auf dem Fußbett befindet, um einen anterio-lateralen Bodenkontaktabschnitt des Fersenbeins auf der Unterseite des anterio-lateralen Fersenbeinablaufs in einem anterio-lateralen Bereich des Fersenbeins zu unterlegen;
wobei der Hohlraum oder die Hohlräume des ersten Bereichs und des zweiten Bereichs sich nicht medial der Längsmittellinie der Ferse kreuzen und mit keinen anderen Ausgleichshohlräume medial der Mittellinie der Ferse, mit Ausnahme von Hohlräumen, die sich auf dem Fußbett befinden, um den medialen Ablauf des Tuber calcanei des Fußes zu unterlegen.

2. Fußbett nach Anspruch 1, wobei das Fußbett eine Vielzahl von getrennten Körpern umfasst.

3. Fußbett nach Anspruch 2, wobei die einzelnen Körper lösbar miteinander verbindbar sind.

4. Fußbett nach Anspruch 1, wobei das Fußbett integral mit einem Schuhwerk ist.

5. Fußbett nach Anspruch 4, wobei das Schuhwerk eine flexible Socke oder einen Strumpf umfasst.

6. Fußbett nach Anspruch 1, wobei das Fußbett konfiguriert ist, um lösbar an einer Sohle eines Fußes befestigbar zu sein.

7. Fußbett nach Anspruch 1, wobei das Fußbett eine Einlegesohle umfasst, die in bereits vorhandenes Schuhwerk einsetzbar ist.

8. ein Fußbett nach Anspruch 1, wobei das Fußbett einen oder mehrere gegebenenfalls herausnehmbare Abschnitte umfasst, um einen oder mehrere Hohlräume zu schaffen.

9. Fußbett nach Anspruch 1, wobei jeder Hohlraum ein Loch durch das Fußbett oder eine Vertiefung im Fußbett umfasst.

10. Fußbett nach Anspruch 9, ferner umfassend eine dünne, flexible Schicht, die sich über jeden Hohlraum erstreckt und konfiguriert ist, um unter dem Gewicht einer auf dem Fußbett stehenden Person in jeden Hohlraum verformbar zu sein.

11. Fußbett, wie es in einem vorangehenden Anspruch, wobei der Hohlraum oder die Hohlräume im ersten und zweiten Bereich durch die Verwendung von teilchenförmigem Material erzeugt werden.

## Revendications

1. Semelle configurée pour supporter un pied et comprenant un vide ou des vides formés dans la semelle, sous la totalité de chacune des régions suivantes (i) et (ii) de la semelle, dans laquelle le ou chaque vide a une taille minimum de 0,6 cm² et est configuré pour que les tissus du pied se déforment dans le ou les vides, **caractérisée en ce que** :
(i) une première région est située sur la semelle pour être située sous une partie latérale d'une partie antéro-médiale du calcanéum du pied lorsqu'il est supporté par la semelle et située dans la partie antérieure du calcanéum, ayant un bord médial le long d'une ligne médiane longitudinale du talon et s'étendant latéralement de la ligne médiane jusqu'à un point qui est sensiblement aligné avec l'aspect latéral de la quatrième phalange ; et
(ii) une seconde région est située sur la semelle pour être située sous une partie antéro-latérale du calcanéum en contact avec le sol, sur la face inférieure du processus calcanéen antéro-latéral, dans une région antéro-latérale du calcanéum ;
dans lequel le ou les vides de la première région et de la seconde région ne se croisent pas médialement par rapport à la ligne médiane longitudinale du talon et sans autre vide d'équilibrage médial de la ligne médiane du talon, excepté pour des vides situés sur la semelle pour être situés sous le processus médial du tubercule du calcanéum du pied.

2. Semelle selon la revendication 1, dans laquelle la semelle comprend une pluralité de corps séparés.

3. Semelle selon la revendication 2, dans laquelle les corps séparés peuvent être connectés de manière détachable les uns aux autres.

4. Semelle selon la revendication 1, dans laquelle la semelle est solidaire d'un article chaussant.

5. Semelle selon la revendication 4, dans laquelle l'article comprend une chaussette souple ou un bas.

6. Semelle selon la revendication 1, dans laquelle la semelle est configurée pour pouvoir être fixée de manière détachable à une plante de pied.

7. Semelle selon la revendication 1, dans laquelle la semelle comprend une première pouvant être insérée dans un article chaussant préexistant.

8. Semelle selon la revendication 1, dans laquelle la semelle comprend une ou plusieurs sections facultativement amovibles afin de créer un ou plusieurs vides.

9. Semelle selon la revendication 1, dans laquelle chaque vide comprend un trou à travers la semelle ou un enfoncement dans la semelle.

10. Semelle selon la revendication 9,
comprenant en outre une couche mince et flexible qui s'étend sur chaque vide et est configurée pour être déformable dans chaque vide sous le poids d'une personne se tenant sur l'assise plantaire.

11. Semelle selon l'une quelconque des revendications précédentes, dans laquelle le ou les vides dans les première et seconde régions sont créés par l'utilisation d'un matériau particulaire.
